# EUROPEAN PATENT APPLICATION

(11) **EP 1 195 192 A1**
(43) Date of publication of application: **10.04.2002**
(21) Application number: 00203435.3
(22) Date of filing: 03.10.2000
(51) Int. Cl.: B01J 19/02, B01J 19/24, C07D 201/08

(54) **Process for the preparation of a mixture of epsilon-caprolactam and/or epsilon-caprolactam precursors**

(71) Applicant: DSM N.V., 6411 TE Heerlen (NL)
(72) Inventor: Guit, Rudolf Philippus Maria, 6228 GP Maastricht (NL); Notten, Mathieu Johannes Guillaume, 6171 AR Stein (NL); Pestman, Robert, 5627 CJ Eindhoven (NL)

(57) **Abstract**

Process for the preparation of a mixture of ε-caprolactam and/or ε-caprolactam precursors starting from 5-formylvalerate ester and/or 5-formylvaleric acid, ammonia and hydrogen in the presence of a hydrogenation catalyst, wherein the process is conducted in a reactor of which the internal wall material is an inert material.

## Description

The invention relates to a process for the preparation of a mixture of ε-caprolactam and ε-caprolactam precursors starting from 5-formylvaleric acid and/or 5-formylvalerate ester, ammonia and hydrogen in the presence of a hydrogenation catalyst.

ε-caprolactam precursors are here defined as 6-aminocaproate ester, 6-aminocaproic acid and 6-aminocaproamide.

Such a process is known from WO-A 9835938. This publication describes a process to continuously prepare an aqueous mixture of ε-caprolactam and ε-caprolactam precursors by continuously contacting methyl-5-formylvalerate with hydrogen and an excess of ammonia in the presence of a ruthenium on titanium oxide carrier catalyst. In the examples, the process is performed in a Hastelloy C reactor vessel.

A disadvantage of this process is that the catalyst activity gradually decreases.

The object of the present invention is to provide a process in which the catalyst remains active over a prolonged period of time.

This object is achieved in that the process is conducted in a reactor of which the internal wall material is an inert material.

We have found that the decrease of the catalyst activity is caused by deposition of corrosion metals, especially molybdenum and tungsten, from the reactor wall on the catalyst.

In one embodiment of the invention, the reactor wall is constructed of the inert material. The inert material is chosen from titanium, zirconium, niobium, tantalum, ferritic stainless steel material or duplex stainless steel material (a steel type with a microstructure consisting of a ferritic matrix with austenitic islands), like for example the commercially available SAF 2205 from Sandvik. Preferably the reactor wall material is a ferritic stainless steel material. Most preferred is a reactor wall material that is made of duplex stainless steel material. Duplex stainless steel is for example described in WO-A-95/00674, herein incorporated by reference. Alternatively, the reactor wall and reactor interior in contact with the reaction mixture is chromated. Chromation is conducted according to known methods of chromating metal surfaces for example using eletrolytic deposition of chrome from chrome salt solution.

In another embodiment of the invention, the internal wall of the reactor is provided with a liner of the inert material. The liner material should be able to sustain the reaction temperatures and reaction pressures. The inert material is then preferably chosen from titanium, zirconium, niobium, tantalum or a polymer like for example polytetrafluoroethime polymer (PTFE) or polyvinylidenefluoropolymer (PVDF). A liner allows the characteristics of the liner in contact with the reaction medium to be independently chosen from the characterisics of the rest of the reactor wall material.

The catalytically active metal in the hydrogenation catalyst is chosen from the Groups 8-10 of the Periodic System of the Elements. Preferably the catalytically active metal is chosen from ruthenium, nickel or cobalt. More preferably the catalytically active metal is ruthenium.

The 5-formylvalerate ester can be obtained by hydroformylation of the corresponding pentenoate as for example described in WO-A-9426688 and WO-A-9518089.

The aldehyde compound can be represented by the following general formula: 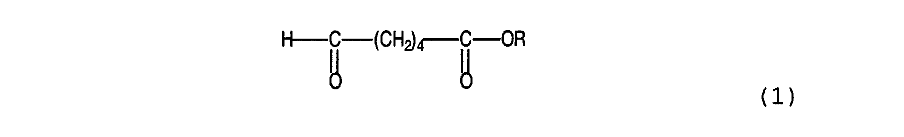 where R is an organic group with 1 to 20 carbon atoms, wherein the organic group is an alkyl, cycloalkyl, aryl or aralkyl group. More preferably R is an alkyl group. Examples of R groups include methyl, ethyl, propyl, isopropyl, n-butyl, tert-butyl, isobutyl, cyclohexyl, benzyl and phenyl. By preference R is methyl or ethyl. Preferably the starting compound is an alkyl 5-formylvalerate because these compounds are more readily available than 5-formylvaleric acid. Unless otherwise stated, reference herein to the formyl-starting compound means alkyl 5-formylvalerate, 5-formylvaleric acid, or both.

When the reductive amination of 5-formylvaleric acid and/or 5-formylvalerate ester is conducted in two steps, i.e. a first step is carried out under "non-hydrogenation conditions" and a second step is carried out under "hydrogenation conditions", like for example described in EP-A-729943. By 'non-hydrogenating conditions' it is understood that the reaction conditions are such that no hydrogen is present or if hydrogen is present, the 5-formylvalerate esters or a reaction product of it is not or virtually not reduced by the hydrogen. In general, non-hydrogenating conditions are achieved by carrying out the first step in the absence of a hydrogenation catalyst. In the second step the term 'hydrogenating conditions' means that the reaction conditions are such that the reaction product(s) obtained in the first step can be reduced by hydrogen. In general hydrogenation conditions are achieved when hydrogen and a hydrogenation catalyst are present. In case the reductive amination is performed in two steps, the second step is performed with the process according to the invention.

The hydrogenation catalyst comprises at least one of the metals of Groups 8-10 of the Periodic System of the Elements (Handbook of Chemistry and Physics, 70th edition, CRC Press, 1989-1990). Preference is given to Ru-, Ni- or Co-containing catalysts. In addition to Ru, Co and/or Ni the cataysts can also contain other metals for example Cu, Fe, Rh, Pt and/or Cr. The catalytically active metals may be applied onto a carrier or not. Suitable carriers are for example aluminium oxide, silica, titanium oxide, zirconium oxide, magnesium oxide and carbon. Titanium oxide is preferably used as the carrier because of its high chemical and mechanical stability and because the selectivity to the preferred (intermediate) compounds is found to be relatively high when this support is used. Preferably anatase is used as titanium oxide. Non-carried metals can be used for example in the form of a finely dispersed suspension for example finely dispersed ruthenium. Preferred Ni- and Co-containing catalysts are Raney nickel and Raney Cobalt optionally in combination with small amounts of another metal, for example Cu, Fe and/or Cr. Most preferred are ruthenium containing catalysts.

A relatively small but catalytically effective amount of the catalyst is used in the present process. The amount of ruthenium (as metal) in the catalyst (metal plus carrier (if present)) is generally between 0.1 and 10 wt.%. The mean particle size (d₅₀) is preferably between 10 and 100 µm, when the catalyst is present as a slurry in the reaction mixture or between 0.001 and 0.05 m, when the catalyst is present in a fixed bed.

Preferably, the process of the invention is performed in the presence of water. The water content in the reaction mixture is preferably at least 10 wt.% and more preferably between about 15 and about 60 wt.% and most preferably between about 20 and about 50 wt.%

If the starting compound is an alkyl 5-formylvalerate it is preferred that some alcohol, corresponding to this alkyl group is present in the reaction mixture. The concentration of the corresponding alcohol can be between 1 and 15 wt.%, although the alcohol concentration is preferably between 5 and 15 wt.% in order to improve the solubility of the alkyl 5-formylvalerate when the concentration of the latter compound is relatively high (> 15 wt.%).

The molar ratio of ammonia and formyl-starting compound in the reductive amination step is preferably between about 3:1 and about 30:1, and more preferably is between about 5:1 and about 20:1.

The temperature is preferably between about 40°C and about 200°C, and more preferably between about 80°C and about 160°C.

The process is preferably conducted under pressure. In general, the pressure is equal or greater than the resulting equilibrium pressure of the liquid reaction mixture employed. The pressure is preferably between 0.5 and 10 MPa.

The molar amount of hydrogen is at least equal to the molar quantity of formyl-starting compound. The molar ratio of hydrogen to the formyl-starting compound is preferably between about 1.00 to about 100.

The present invention can be performed continuously in a fixed bed reactor in which the heterogeneous hydrogenation catalyst is present. An advantage of this reactor is that the reactants are easily separated from the hydrogenation catalyst. Another manner of performing the reductive amination is by way of one or more continuously operated well mixed contactors in series in which the hydrogenation catalyst is present as a slurry (slurry reactor). This manner of operation has the advantage that the heat of the reaction can be easily controlled by, for example, a cooled feed or by way of internally placed cooling devices. Examples of specific and suitable slurry reactors are one or multiple staged bubble columns or a gas lift-loop reactor or a continuously stirred tank reactor (CSTR). The slurry-hydrogenation catalyst can be separated from the reaction mixture by for example using hydrocyclones and/or by filtration, for example by cake- or cross-flow filtration.

The catalyst concentration can be suitably selected across a wide concentration range. In a fixed bed reactor the amount of catalyst per reactor volume will be high, while in a slurry-reactor this concentration will, in general be lower. In a continuously operated slurry reactor the weight fraction of catalyst (including the carrier) is typically between about 0.1 and about 30 weight % relative to the total reactor content.

### Example I

A continuous reductive amination experiment was conducted in a Hastelloy C microreactor with a liquid volume of 25 ml which had been chromated (1 gram of 1.75 wt% ruthenium on titanium oxide was introduced in the reactor). An aqueous stream consisting of 40 wt% NH₃, 25 wt% methyl-5-formylvalerate and 7 wt% methanol in water was continuously fed to the reactor. The reaction was performed at a temperature of 140°C and a pressure of 4 MPa. The residence time was 1 hour.

After the experiment the metal content of the catalyst and of the reaction mixture was measured using Neutron Activation Analysis (NAA) with short and long living radioisotopes using the k₀ standardization method.

The results of Example I are given in figure 1 and in tables 1 and 2.

### Comparative experiment A

Example I was repeated with a Hastelloy C microreactor on which no chromation treatment was executed. The results of this experiment are given in figure 1 and in tables 1 and 2.

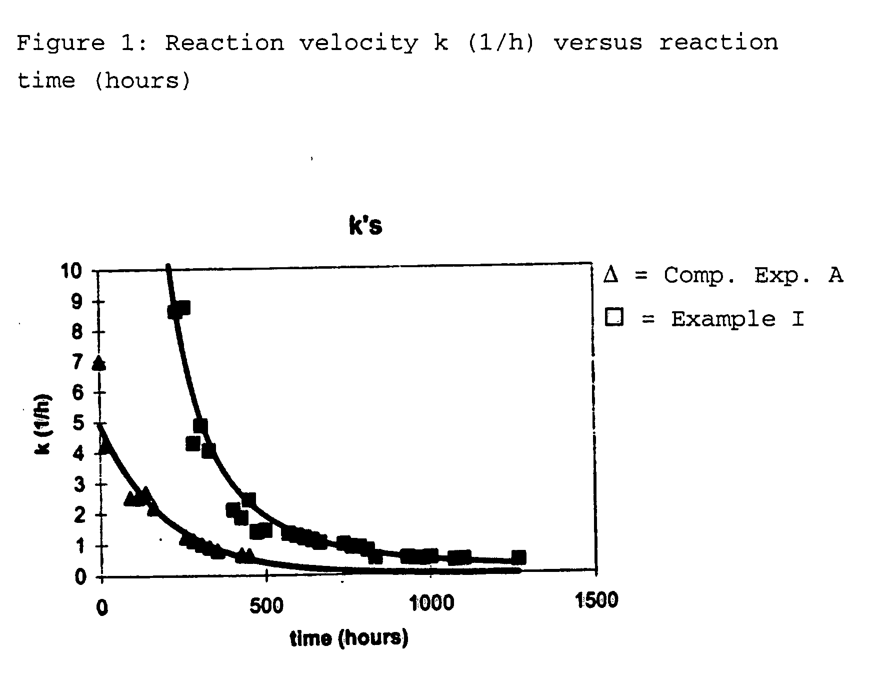

From figure 1 it can be seen that the deactivation in Comparative Experiment A is faster than in Example I.

**Table 1:**

| Metal analysis of catalyst | | | |
|---|---|---|---|
| | | Comp. Ex. A | Exp. I |
| Reactor | | Hastelloy C | Chromated Hastelloy C |
| Ru | Wt.% | 1.63 | 1.57 |
| Cr | mg/kg | 2600 | 2260 |
| Ni | mg/kg | 700 | 410 |
| Cu | mg/kg | 310 | 340 |
| Mo | mg/kg | 2320 | 1020 |
| W | mg/kg | 520 | 470 |
| Run time | hours | 423 | 1272 |

From table 1, it can be seen that the amount of corrosion metals deposited on the catalyst in Comparative Experiment A is about equal to - and for molybdenum even twice - the amount of corrosion metals deposited on the catalyst in Example I, even though the catalyst in Comparative Experiment A was used only during 423 hours while the catalyst in Example I was used during 1272 hours.

**Table 2:**

| Metal analysis of reductive amination product and reaction velocity k (1/h) at start, at half the experiment and at the end of the experiment | | | | |
|---|---|---|---|---|
| Example I | | | | |
| | | Start | middle | end |
| | | | | |
| Ru | mg/kg | 0.13 | 0.03 | 0.01 |
| Cr | mg/kg | 0.67 | 0.44 | 0.34 |
| Ni | mg/kg | 1.28 | 0.80 | 0.78 |
| Cu | mg/kg | - | 0.04 | - |
| Mo | mg/kg | 0.10 | 0.15 | 0.16 |
| W | mg/kg | 0.01 | 0.02 | 0.02 |
| k(1/h) | | 9 | 1.1 | 0.5 |

| Comparative experiment A | | | | |
|---|---|---|---|---|
| | | Start | middle | end |
| | | | | |
| Ru | mg/kg | 0.17 | 0.07 | 0.04 |
| Cr | mg/kg | 1.33 | 0.91 | 0.99 |
| Ni | mg/kg | 2.50 | 1.80 | 1.75 |
| Cu | mg/kg | - | 0.03 | 0.04 |
| Mo | mg/kg | 0.29 | 0.26 | 0.30 |
| W | mg/kg | 0.02 | 0.02 | 0.03 |
| | | | | |
| k(1/h) | | 4.1 | 2.0 | 0.8 |

From table 2, it can be seen that in Example I the corrosion metals are present in lower concentrations than in Comparative Experiment A.

## Claims

1. Process for the preparation of a mixture of ε-caprolactam and/or ε-caprolactam precursors starting from 5-formylvaleric acid and/or 5-formylvalerate ester, ammonia and hydrogen in the presence of a hydrogenation catalyst, wherein the process is conducted in a reactor of which the internal wall material is an inert material.

2. Process according to claim 1, wherein the reactor wall is constructed of the inert material.

3. Process according to claim 2, wherein the inert material is chosen from titanium, zirconium, niobium, tantalum, ferritic stainless steel material or duplex stainless steel material.

4. Process according to any one of claims 2-3, wherein the reactor wall material is a duplex stainless steel material.

5. Process according to claim 1, wherein the internal wall of the reactor is provided with a liner of the inert material.

6. Process according to claim 5, wherein the inert material is chosen from titanium, zirconium, niobium, tantalum, polytetrafluoroethylene or polyvinylidenefluoropolymer.

7. Process according to any one of claims 1-6, wherein the hydrogenation catalyst contains at least one Group 8-10 element of the Periodic system of the Elements as catalytically active metal.

8. Process according to Claim 7, wherein the catalytically active metal is chosen from ruthenium, nickel or cobalt.

9. Process according to Claim 7, wherein the catalytically active metal is ruthenium.

10. Reaction mixture containing ε-caprolactam and/or ε-caprolactam precursors prepared according to one of the claims 1-9, wherein the reaction mixture contains substantially no molybdenum and/or tungsten.
